(19) Europäisches Patentamt
European Patent Office
Office européen des brevets

(11) Publication number: **0 081 959**
**A2**

(12) # EUROPEAN PATENT APPLICATION

(21) Application number: **82306466.2**

(22) Date of filing: **06.12.82**

(51) Int. Cl.³: **C 07 D 275/06**, C 07 F 5/06, A 23 G 3/30

(30) Priority: **11.12.81 US 329644**

(43) Date of publication of application: **22.06.83**
**Bulletin 83/25**

(84) Designated Contracting States: **BE CH DE FR GB LI SE**

(71) Applicant: **WARNER-LAMBERT COMPANY, 201 Tabor Road, Morris Plains New Jersey 07950 (US)**

(72) Inventor: **Rieger, Martin Max, 304 Mountain Way, Morris Plains New Jersey 07950 (US)**
Inventor: **Yang, Robert Kuo-How, 90 Sun Valley Way, Morris Plains New Jersey 07950 (US)**

(74) Representative: **Jones, Michael Raymond et al, Haseltine Lake & Co. 28 Southampton Buildings Chancery Lane, London WC2A 1AT (GB)**

(54) **An aluminium salt of saccharin, and chewing gum compositions containing that salt.**

(57) An aluminium salt of saccharin and a process for its preparation are disclosed. Chewing gum compositions incorporating an aluminium salt of saccharin are disclosed and have improved sweetness retention.

EP 0 081 959 A2

"AN ALUMINIUM SALT OF SACCHARIN, AND CHEWING GUM COMPOSITIONS CONTAINING THAT SALT"

Chewing gums which contain saccharin either alone or in combination with other natural or artificial sweeteners are well known. The saccharin is usually present in the chewing gum formulation in the form of a water-soluble salt, e.g. the sodium salt. Upon mastication of such a chewing gum which contains a water-soluble saccharin salt, one perceives an initial burst of sweetness which rapidly declines and is soon noticed to disappear. This leaves a piece of chewing gum which is no longer sweet and which is usually disposed of in short order by the user. In order to prolong the sweetening effect of saccharin, it has been proposed to utilize saccharin in a less soluble form. The use of one such form, the free acid form of saccharin, has met with only limited success as this material is still too water-soluble and is, therefore, rapidly lost from the bolus during mastication. We have now discovered, and this forms the basis of the present invention, that chewing gums comprising the aluminium salt of saccharin possess an unexpectedly prolonged period of sweetness retention when

compared to similar chewing gum formulations containing other forms of saccharin.

According to one aspect of the present invention, there is provided an aluminium salt of saccharin.

Another aspect of the present invention provides a chewing gum composition which includes, as a sweetening agent, an aluminium salt of saccharin.

A further aspect of the present invention is a process for producing an artificially sweetened chewing gum composition having prolonged sweetness retention, which process comprises incorporating an aluminium salt of saccharin in the chewing gum to sweeten the same.

Yet another aspect of the present invention provides a process for preparing the aluminium salt of saccharin, which comprises reacting the free acid form of saccharin with an aluminium alkoxide.

Many salts of saccharin are known; however, no reference has been found which describes the aluminium salt of saccharin.

The aluminium salt of saccharin may be produced byany one of several procedures. In one such procedure, the free acid form of saccharin is contacted with an aluminium alkoxide in a suitable non-reactive solvent. The aluminium salt is thereafter isolated and purified by standard procedures. Any aluminium alkoxide may be utilized, but aluminium isopropoxide is preferred. In a preferred process of preparation, the aluminium isopropoxide is dissolved in hot isopropyl alcohol, the solution is filtered to remove any insolubles and is then added directly to a solution of the free acid form of saccharin in isopropyl alcohol. The combined solution is refluxed, during which time a white precipitate is formed. The solution

is allowed to cool and the preciptate is collected by filtration. The precipitate is washed with isopropyl alcohol and subsequently dried in vacuo at elevated temperature. The aluminium salt of saccharin, so produced, is used in producing the chewing gum composition of the present invention and may also be used for other purposes in which slow and continuous release of sweetness is desirable.

The alkoxide moiety of the aluminium alkoxides contemplated by the present invention includes those having carbon chains of from 1 to 6 carbon atoms. The chains may be either straight or branched; examples of such alkoxides are methoxide, ethoxide, isopropoxide and hexoxide.

In general, the chewing gum compositions of the present invention may be produced using generally standard ingredients and standard procedures and processing equipment. Since the aluminium salt of saccharin is novel, it, of course, cannot be considered a standard chewing gum ingredient. The technique of incorporating this novel ingredient in chewing gum formulations, however, is within the skill of the art. Thus, the aluminium salt of saccharin may be added to a standard chewing gum composition in an amount sufficient to provide an acceptable sweet chewing gum. This amount is readily ascertained by those skilled in the art. The aluminium salt of saccharin may be utilized alone or in combination with other known natural and artificial sweeteners.

The chewing gum compositions comprising the aluminium salt of saccharin unexpectedly have been found to possess a prolonged sweetness retention when compared to prior art chewing gum formulations which do not contain this novel ingredient. The prolonged sweetness retention

is demonstrated by the following procedure.

Six subjects chewed each of the two chewing gum compositions described below (the details of the preparation of the two compositions being given at the end of the description):

| | Control | Test |
|---|---|---|
| Gum base | 26.4% | 26.4% |
| Glycerin USP | 5.0% | 5.0% |
| Water, Potable | 2.7% | 2.7% |
| Sorbitol 712 | 64.6% | 64.6% |
| Al-Saccharin Salt | - | 0.12% |
| Saccharin, Free Acid Form | 0.1% | - |
| Flavour | 1.2% | 1.2% |

The sweetness perception during chewing of the control batch lasted for 11 minutes while that of the test batch lasted for 32 minutes.

All types of chewing gums are contemplated by the invention; thus, stick, shredded, slab and chunk gums are contemplated. Regular chewing gum, bubble gum, hard-coated gum and centre-filled gums are also included. The aluminium salt of saccharin may be incorporated in the chewing gum base, may be added to the surface of the chewing gum piece as a dusting or as a part of the normal surface dusting, may be incorporated in the hard coating of a hard coated gum, or may be included in the centre fill mix of a centre filled gum.

The use of the aluminium salt of saccharin is not limited to chewing gums. Thus, other applications are also contemplated by the present invention. Examples of such applications are pressed mints, pressed candies and boiled candies.

Solvates of the aluminium salt of saccharin with non-toxic solvents are also contemplated by the present invention. Thus, for example, the hydrate and the ethanolate are contemplated.

Processes for preparing the aluminium salt of saccharin are illustrated by the following examples. Other processes for preparing this novel salt are within the skill of the art and are contemplated by the present invention.

EXAMPLE 1

2.04 Grams of aluminium isopropoxide were dissolved in 100 ml of hot isopropanol and filtered to remove any insolubles. The filtrate was slowly added to 5.70 grams of saccharin acid dissolved in 200 ml of isopropanol and refluxed for 30 minutes during which time an insoluble, white product was formed. After cooling and filtration, the filter cake was washed with isopropanol. The product was air dried. The yield was 3 grams of the aluminium salt of saccharin.

EXAMPLE 2

The process of preparation was much the same as that in Example 1, except that 6.84 grams of aluminium isopropoxide in 200 ml of ispropanol were reacted with 11.0 grams of saccharin in 300 ml of isopropanol. The yield was 7.7 grams of the aluminium salt of saccharin, as dried in air.

EXAMPLE 3

The process of preparation was much the same as that in Example 1, except that 10.0 grams of aluminium isopropoxide were reacted with 11 grams of saccharin. After air drying, the yield was 9.5 grams of the aluminium salt of saccharin.

EXAMPLE 4

Aluminium isopropoxide (20 grams) was dissolved in 200 ml of dry toluene and filtered to remove any insolubles. The filtrate was slowly added to 10.0 grams of saccharin and dissolved in 300 ml of isopropanol, and the resulting mixture was refluxed for 30 minutes. No precipitate

was formed immediately as in Examples 1, 2 and 3. After standing at room temperature for several days, a white precipitate did form. The precipitate was filtered and washed with toluene. The product was first dried in air and then at 110°C in vacuum for four hours. Yield 10.5 grams of the aluminium salt of saccharin.

The aluminium salts of saccharin obtained in each of the above examples all had essentially identical infrared spectra and upon analysis were found to contain 64 to 76.5 percent saccharin (computed as the acid) and 9.4 to 10.4 percent aluminium. This composition did not depend on the ratio of the reactants saccharin and aluminium isopropoxide.

Details of the chemical analyses of the materials isolated in Examples 1 to 4 follow. After brief hydrolysis of the salt in dilute acid, suspensions of the solids were analyzed for their aluminium content by atomic absorption and for their saccharin content by HPLC with the following results:

| Solid from Example | % Aluminium | % Saccharin |
|---|---|---|
| 1 | 10.0 | 64.0 |
| 2 | 9.8 | 67.9 |
| 3 | 9.6 | 66.4 |
| 4 (air dried only) | 9.4 | 72.5 |
| 4 (dried at 110°C in vacuum) | 10.4 | 76.5 |

Based on these analyses it was concluded that the prepared solids conformed to the following structure but were contaminated with small amounts of aluminium hydroxide, isopropanol, and/or water:

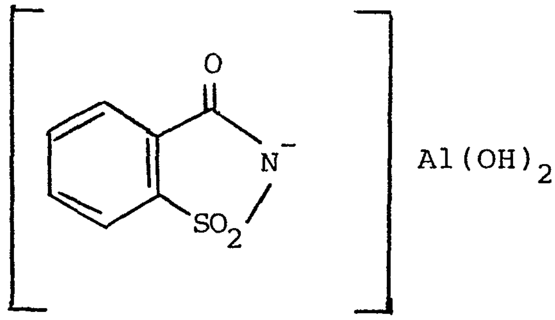

which would show the following theoretical compositions:

| | |
|---|---|
| Aluminium | 11.1% |
| Saccharin | 74.9% |
| OH | 14.0% |

Substantiation of this structure - which however is not essential to the present invention - was obtained as follows:

1. The IR spectra of the compounds prepared in Examples 1 to 4 (KBr pellet) showed a large peak at 3450 $cm^{-1}$ due to OH stretching. Regardless of how the samples were dried or of the severity of the drying conditions (as high as 110°C under vacuum for 4 days), the intensity of this OH peak relative to the other major peaks remained unchanged. It is apparent, therefore, that the OH moiety is an integral part of the molecule which could arise by hydrolysis of the aluminium isopropoxy groups which are contained in a presumed intermediate.

2. In order to confirm the presence of these OH - moieties within the molecule, NMR spectroscopy was conducted on the solid from Example 4 and it was found that the proton chemical shift positions did not correspond to free water but only to the presence of associated OH - groupings.

3. In order to understand more fully the importance of water in the preparation of the aluminium saccharin salt, the following experiment

was conducted:

The following mixture of reagents was added to each of four oven-dried 500 ml glass stoppered volumetric flasks A, B, C and D: Aluminium isopropoxide (5 g) dissolved in 100 ml of isopropanol and dried (under vacuum at 110°C for 18 hours), saccharinic acid (13.5 g) dissolved in 250 ml of dried (with anhydrous $MgSO_4$ overnight) isopropanol. This blend was heated for 30 minutes. Dried (by boiling for 10 minutes) toluene was added to each flask to make 500 ml. Finally distilled water was added as follows:

Flask A ---- no water
Flask B ---- 0.09 ml
Flask C ---- 0.18 ml
Flask D ---- 0.27 ml

Within 12 hours, a large amount of white precipitate had formed in Flask D. Flask C contained a modest amount of precipitate plus some crystalline material, while Flask B contained very little of this white precipitate. On the other hand, Flask A remained clear.

The absence of any precipitate in Flask A is a clear indication that the presumed intermediate is soluble in the solvent system employed. Only in the presence of small amounts of water which may have been inadvertently absorbed during the preparations of Examples 1 to 4 is the aluminium salt of saccharin formed. Since aluminium isopropoxide is known to react readily with small amounts of water to form aluminium hydroxide, it was important to determine the saccharin content of the solids isolated from each of the flasks. Not unexpectedly, all solids isolated contained essentially the theoretically required amount of saccharin:

| Samples | % Aluminium | % Saccharin |
|---|---|---|
| Flask B | 8.3 | 69.9 |
| Flask C - white pot | 10.6 | 67.3 |
| Flask C - crystalline solids | 9.4 | 73.1 |
| Flask D | 11.6 | 66.3 |
| Theoretical Amount | 11.1 | 74.9 |

4. The samples prepared in Examples 1 to 4 were subjected to I.R. spectroscopy. All I.R. spectra showed remarkably similar patterns, even though the mole ratios of the starting materials were different. Especially noteworthy is the region of 1640-1570 $cm^{-1}$ in which a strong doublet peak corresponding to 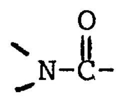 stretching was found. This doublet is absent in the saccharinic acid starting material and indicates that a new chemical compound was formed during the reaction.

5. Finally, x-ray diffraction patterns of aluminium isopropoxide, of saccharinic acid, and the reaction product from Example 4 were compared. The diffraction pattern of the reaction product from Example 4 was shown to be entirely different from the patterns obtained from the starting materials. It is concluded therefrom that the aluminium saccharinate is a new chemical entity.

Preparation of a chewing gum containing the aluminium salt of saccharin of the invention (the Test Sample): In a preheated mixer (50°C), sorbitol powder was first added and mixed for 2 minutes in order to break up any lumps. In a separate container, gum base was melted with moderate heating (90-100°C), and the required amount of aluminium saccharin salt was added and well blended. The molten gum base - aluminium saccharin salt mixture was then added to the

mixer. Next, the glycerin and water were added and mixed for 5 minutes. The heat was turned off, the flavour was added, and the mass was mixed for an additional 3 minutes. The mass was removed from the mixer, allowed to cool, rolled and cut into pieces using mannitol as a dusting medium.

The Control Sample was prepared in exactly the same manner with the exception that the aluminium saccharin was replaced by saccharinic acid.

In order to demonstrate that the loss of saccharin from the Test Samples is significantly slower than that from the Control Sample, chew-out tests were performed as follows: The saccharin content of pieces of gum was determined and then other samples of the gums were chewed by a group of subjects for 10 minutes. At this time, the boluses were removed from the mouth and assayed for their saccahrin contents.

The results tabulated below show that the Test Samples retained almost 50% of their saccharin content after 10 minutes of chewing, while the Control Samples had lost about 75% of their saccharin content. The loss of the sodium salt of saccharin (included for additional comparison) was almost 100%.

| Sample | Saccharin % Chewed Out |
|---|---|
| Control Gum | 75% |
| Test Gum | 51% |
| Commercial Gum with (Na-saccharinate) | 96% |

CLAIMS:

1. An aluminium salt of saccharin.

2. An aluminium salt of saccharin which has the following percentage composition ranges:

Aluminium, 9.4 to 11.1;
Saccharin (as the acid), 64.0 to 74.9;
and
OH, 26.6 to 14.

3. An aluminium salt of saccharin which has the following characteristics on infrared analysis (KBr pellet):

large peak at 3450 $cm^{-1}$; and
strong doublet at 1640-1570 $cm^{-1}$.

4, An aluminium salt of saccharin which has the following formula:-

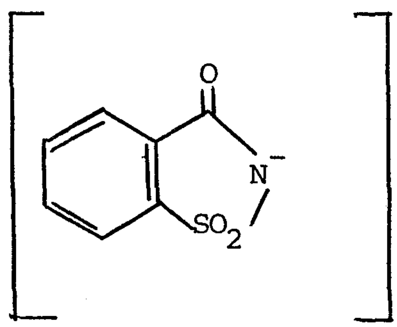

$Al(OH)_2$

5. A process for producing an aluminium salt of saccharin which comprises reacting the free acid form of saccharin with an aluminium alkoxide.

6. A process according to Claim 5, wherein the aluminium alkoxide is aluminium isopropoxide.

7. A chewing gum composition which comprises an aluminium salt of saccharin as claimed in any one of Claims 1 to 4.

8. A composition according to Claim 6 which contains an additional sweetner.

9. A process for producing an artificially sweetened chewing gum composition having prolonged

sweetness retention, which process comprises incorporating an aluminium salt of saccharin in the composition.